Europäisches Patentamt

European Patent Office

Office européen des brevets

(11) Publication number: **0 471 100 A1**

## EUROPEAN PATENT APPLICATION

(21) Application number: **90115559.8**

(22) Date of filing: **14.08.90**

(51) Int. Cl.⁵: **A61K 33/00, A61K 9/22**

(43) Date of publication of application:
**19.02.92 Bulletin 92/08**

(84) Designated Contracting States:
**AT BE CH DE ES FR GB GR IT LI NL SE**

(71) Applicant: **Müderrisoglu, Ali**
**Evrenoszade Sokak 4, Bomonti-Sisli**
**TR-80223 Istanbul(TR)**
Applicant: **Capan, Yilmaz, Prof.**
**Evrenoszade Sokak 4, Bomonti-Sisli**
**TR-80223 Istanbul(TR)**
Applicant: **Hincal, Atilla A., Prof. Dr.**
**Evrenoszade Sokak 4, Bomonti-Sisli**
**TR-80223 Istanbul(TR)**
Applicant: **Ciftci, Kadriye**
**Evrenoszade Sokak 4, Bomonti-Sisli**
**TR-80223 Istanbul(TR)**

(72) Inventor: **Müderrisoglu, Ali**
**Evrenoszade Sokak 4, Bomonti-Sisli**
**TR-80223 Istanbul(TR)**
Inventor: **Capan, Yilmaz, Prof.**
**Evrenoszade Sokak 4, Bomonti-Sisli**
**TR-80223 Istanbul(TR)**
Inventor: **Hincal, Atilla A., Prof. Dr.**
**Evrenoszade Sokak 4, Bomonti-Sisli**
**TR-80223 Istanbul(TR)**
Inventor: **Ciftci, Kadriye**
**Evrenoszade Sokak 4, Bomonti-Sisli**
**TR-80223 Istanbul(TR)**

(74) Representative: **Dickel, Klaus, Dipl.-Ing.**
**Julius-Kreis-Strasse 33**
**W-8000 München 60(DE)**

(54) **Sustained release lithium carbonate tablets and method for preparing the same.**

(57) Sustained release lithium carbonate tablets comprising 50 to 70 % lithium carbonate, 20 to 25 % lactose, 5 to 15 % carbomer and 0.5 to 2 % magnesium stearate show considerably reduced high peak blood levels avoiding gastrointestinal side effects of lithium and providing for extended dosing periods.

EP 0 471 100 A1

The present invention relates to sustained release lithium carbonate tablets and a method for preparing the same.

Lithium carbonate is widely used in the treatment of mania and in preventing the recurrence of both manic and depressive symptoms. The therapeutic index of the drug is narrow, and side effects are not uncommonly seen in patients whose serum lithium levels are maintained within the therapeutic range. Conventional lithium carbonate tablets make the drug immediately available for rapid absorption and relatively high peak blood levels. Adverse side effects are commonly associated with high lithium serum concentrations. There are high absorption peaks increasing the gastrointestinal side effects. Depending on the rapid lithium availability of the conventional commercially available lithium carbonate tablets dosing three or four times daily is required.

In view of these shortcomings of conventional lithium carbonate tablets it is an object of the present invention to provide for sustained release lithium carbonate tablets reducing the absorption peaks and the gastrointestinal side effects of lithium.

The sustained release lithium carbonate tablets according to the invention comprise 50 to 70 %, preferrably 67 % lithium carbonate, 20 to 25 %, preferrably 22 % lactose, 5 to 15 %, preferrably 10 % carbomer and 0.5 to 2 %, preferrably 1 % magnesium stearate.

The sustained release lithium carbonates according to the invention produce a much smoother curve of release than conventional tablets. The amplitude of the postabsorptive plasma lithium level is decreased and thus also the fluctuations experienced during a single dose interval. Dosing can be reduced to about two times daily.

The tablets are prepared by blending lithium carbonate, lactose and carbomer for 20 to 30 minutes, preferrably 25 minutes, screening in the magnesium stearate and blending for additional 5 minutes. Thereafter the blended material is compressed into tablets having a hardness in the range of 7 to 9, preferrably 7.3 to 8 kg on a Heberlein hardness tester.

The invention is further explained by the following examples and tests.

There have been prepared tablets having the following formulation:

| Lithium carbonate | 400 mg |
| Carbomer | 60 mg |
| Lactose | 134 mg |
| Magnesium stearate | 6 mg |

Carbomer was used as matrix material and lactose as a filler. In addition,carbomer is used in a range of 5 to 15 %, preferrably 10 % of the total tablet weight.

The powders were mixed and directly compressed with 1 % of magnesium stearate incorporated as a lubricant prior to compression. The compression was carried out on a single-punch tablet machine at a tablet weight of 600 mg using a flat, non-beveled punch of 12 mm diameter and the tablet hardness was kept constant within the range of 7.5 to 8 kg on a Heberlein hardness tester. Time and temperature of the manufacuring process did not appear to effect the tablet characteristics.

The dissolution rate in vitro of the dosage form was measured by the USP Paddle Method at 100 rpm in 900 ml of distilled water at 37°C.

20 % by weight lithium carbonate is released after 2 h, 44 % by weight lithium carbonate is released at 4 h, 72 % by weight lithium carbonate is released after 6 h, and 84 % by weight lithium carbonate is released after 8 h.

Quantitatively, the oral solution of lithium carbonate produced about 2.2 times more fluctuation in serum lithium values obtained in twelve he healthy subjects than sustained release tablets.

In Vivo Studies

Twelve adults, 4 women and 8 men, between the age of 23 and 47 years (29.5 ± 11.26 (S.D.)) and with body weights between 56 and 74 kg (64.6 ± 6.03 (S.D.)) volunteered for this investigation. Initially they were divided into two groups of 6 subjects. One group received two 400 mg sustained release (LIT-O-DUR) tablets and the other received three 300 mg conventional release tablets of lithium carbonate with 250 ml of water.

Before drug administration a 5 ml blood sample was collected from each subject.

Results:

As expected, the lithium carbonate was released from tablets more slowly with an increase in carbomer content. The main objective was to have a release in the range of 25 to 40 % by 2 hours, 40 to 60 % by 4 hours and 70 to 90 % by 8 hours for the 12 hour sustained release preparations. The 10 % carbomer matrix tablets LIT-O-DUR gave desired release to be promising for a 12 hour sustained release tablet.

In vitro and in vivo release studies show that carbomer is the ingredient that is controlling the release rate. Lactose is only a filler excipient in this formulation.

The formulated sustained tablets LIT-O-DUR produced a much smoother curve than the conventional release tablet of lithium carbonate. The sustained release LIT-O-DUR tables had a significantly lower $C_{max}$ - (maximum serum concentration) and a later $t_{max}$ (time to reach $C_{max}$). It is of interest that the fluctuation in serum lithium level

during the absorptive phase was lower when administering the sustained release LIT-O-DUR tablets than when administering the conventional ones. This is important since the sustained release LIT-O-DUR tablets, containing lithium carbonate in an hydrophylic matrix (10 % carbomer), may afford the possibility of more uniform absorption characteristics and may reduce the incidence of side effects that usually accompany the lithium therapy and which are considered to follow the absorptive serum high peak.

### Claims

1. Sustained release lithium carbonate tablets comprising 50 to 70 % lithium carbonate, 20 to 25 % lactose, 5 10 15 % carbomer, 0.5 to 2 % magnesium stearate.

2. Sustained release lithium carbonate tablets according to claim 1, comprising 67 % lithium carbonate, 22 % lactose, 10 % carbomer, 1 % stearate.

3. Method of preparing sustained released lithium carbonate tablets by blending lithium carbonate, lactose and carbomer for 20 to 30 minutes, screening in the magnesium stearate and blending for additional 5 minutes, therafter compressing the blended material into tablets having a hardness in the range of 7 to 9 kg on a Heberlein hardness tester.

4. Method according to claim 3, according whereto the lithium carbonate, lactose and carbomer are blended for 25 minutes and the tablet hardness is kept in the range of 7.5 to 8 kg on a Heberlein hardness tester.

European
Patent Office

EUROPEAN SEARCH
REPORT

Application Number

EP 90 11 5559

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CONGR. INT. TECHNOL. PHARM., 5TH, vol. 4, 1989, pages 110-117; Y. CAPAN et al.: "Influence of filler excipients on the release rate of sustained-release lithium carbonate tablets" <br> * Whole document * <br> – – – | 1-4 | A 61 K 33/00 <br> A 61 K 9/22 |
| X | PHARMACEUTICAL RESEARCH, vol. 7, no. 4, 1990, pages 359-363; K. CIFTCI et al.: "Formulation and in vitro-in vivo evaluation of substained-release lithium carbonate tablets" <br> * Whole document * <br> – – – – – | 1-4 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 19 April 91 | HOFF P.J.L. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document